# EUROPÄISCHE PATENTSCHRIFT

(11) **EP 1 037 085 B1**
(45) Veröffentlichungstag und Bekanntmachung des Hinweises auf die Patenterteilung: **09.05.2007**
(21) Anmeldenummer: 99104899.2
(22) Anmeldetag: 11.03.1999
(51) Int. Cl.: G02B 7/00, A61B 19/00, F16M 11/04

(54) **Aufhängung für ein Operationsmikroskop**
Suspension system for surgical microscope
Suspension pour un microscope chirugical

(43) Veröffentlichungstag der Anmeldung: 20.09.2000
(73) Patentinhaber: Möller-Wedel GmbH, 22880 Wedel (DE)
(72) Erfinder: Duis, Wilhelm, 25491 Hetlingen (DE)
(74) Vertreter: Glawe, Delfs, Moll

(56) Entgegenhaltungen:
- EP-A- 0 023 003
- WO-A-98/52484
- DE-A- 4 233 658
- GB-A- 1 400 639
- US-A- 5 345 087

## Beschreibung

Die Erfindung betrifft eine Aufhängung für ein Operationsmikroskop mit einem Aufhängearm und einem Drehlager mit im wesentlichen horizontaler Drehachse, die einen ersten Arm aufweist, der über das Drehlager mit dem Aufhängearm und über einen zweiten Arm, der mit dem ersten Arm über ein erstes Schwenklager verbunden ist, dessen Achse im wesentlichen parallel zur Drehachse ist, mit dem Operationsmikroskop verbunden ist, wobei eine erste Feststelleinrichtung zur winkelmäßigen Arretierung der Arme relativ zueinander vorgesehen ist, wobei der zweite Arm ein zweites Schwenklager aufweist, über das er mit einem dritten Arm verbunden ist, der mit dem Mikroskop verbunden ist, wobei die Achsen des Drehlagers und der ersten und zweiten Schwenklager im wesentlichen parallel zueinander angeordnet sind und wobei eine zweite Feststelleinrichtung zur winkelmäßigen Arretierung der zweiten und dritten Arme aneinander vorgesehen ist.

In der Mikrochirurgie kleiner biologischer Strukturen, z.B. in der Neurochirurgie, HNO-Chirurgie und der Dentalchirurgie, werden seit Jahrzehnten Operationsmikroskope eingesetzt, die vielfach mit wechselnder peripherer Ausstattung ausgerüstet sind. So können je nach Anforderung der speziellen Operation Kameras oder Mitbeobachtungstuben hinzugefügt oder entfernt werden.

Gleichzeitig erfordern zahlreiche Operationen eine leichte Beweglichkeit des Operationsmikroskops, damit dessen Blickachse während des Operationsverlaufs mit möglichst geringer Kraftanstrengung verändert werden kann. In der Regel werden dafür mechanische oder elektrische Bremsen an den Achsen von Stativ und Mikroskopaufhängung angebracht, nach deren Lösen das Operationsmikroskop in bis zu sechs Koordinaten (drei Ortskoordinaten und drei Drehwinkel) frei beweglich ist. Dabei muss das Gesamtsystem so gut gewichtsausgeglichen sein, dass beim Lösen der Bremsen das Operationsmikroskop weder als ganzes in Bewegung kommt noch eine ungewollte Drehung ausführt. Ersteres wird durch einstellbare Federkräfte oder Gegengewichte am Stativ erreicht, letzteres dagegen erfordert, dass die Drehmomente des Mikroskopkörpers durch Federkräfte ausgeglichen werden oder der Schnittpunkt der Drehachsen und der Schwerpunkt zusammenfallen.

Es gibt Operationsmikroskope, bei denen der Ausgleich der Drehmomente am Mikroskopkörper durch Federkräfte erfolgt. Diese Bauweise ist jedoch nachteilig, da die Federkraft nach Austausch von peripherer Ausstattung durch Probieren neu eingestellt werden muss. Dieser Prozess ist zeitraubend und kann zu Fehlern führen. Außerdem ändern sich die Kräfte mit der Drehung, so dass ein guter Ausgleich bestenfalls für kleine Drehwinkel erreicht werden kann.

Andere Operationsmikroskope auf dem Markt beinhalten Kreuzschlitten, mit denen die Drehachsen so gelegt werden können, dass sie sich im Schwerpunkt des Mikroskops treffen. Nachteilig daran ist jedoch, dass ein Abgleich ebenfalls nur durch Probieren erfolgen kann und damit fehlerbehaftet ist. Zudem liegt der Schwerpunkt meist so nahe an den Okularen, dass der Kreuzschlittenmechanismus den Chirurgen behindert. Gegengewichte, die den Schwerpunkt vom Chirurgen entfernen, können eine gewisse Abhilfe bringen, machen das Operationsmikroskop aber schwer und unhandlich und sind daher ebenfalls nachteilig.

Bei der eingangs-genannten Aufhängung eines Operationsmikroskops (GB 1 400 639) erfolgt der Gewichtsausgleich mit zwei Gewichten, was die oben im Zusammenhang mit Gegengewichten erwähnten zusätzlichen Maßnahmen erfordert.

Die Aufgabe der Erfindung besteht darin, eine Aufhängung für ein Operationsmikroskop zu schaffen, die einfach aufgebaut ist und einfach und schnell gewichtsmäßig abzugleichen ist.

Die erfindungsgemäße Lösung besteht darin, dass der zweite Arm derart scheibenförmig oder abgewinkelt ausgebildet ist, dass die Achsen des Drehlagers und der ersten und zweiten Schwenklager an den Ecken eines im Wesentlichen rechtwinkligen Dreiecks mit der Achse des Drehlagers beim rechten Winkel angeordnet sind.

Um die Aufhängung gewichtsmäßig abzugleichen, nachdem das Mikroskop angebracht ist oder aber Zubehörteile hinzugefügt oder weggenommen worden sind, wird der bewegliche Teil der Aufhängung so gedreht, dass sich das erste Schwenklager vertikal über der Achse des Drehlagers befindet. Die erste Feststelleinrichtung wird dann gelöst, so dass sich das Mikroskop mit dem zweiten Arm so einpendelt, dass sich der Schwerpunkt des beweglichen Teils vertikal unterhalb der Achse des ersten Schwenklagers, d. h. auf der Verlängerung der Verbindungslinie vom ersten Schwenklager zur Achse des Drehlagers befindet. Der Schwerpunkt ist auf diese Weise näher am ersten Drehlager, als dies vorher der Fall war. In dieser Stellung werden die ersten und zweiten Arme mit der ersten Feststelleinrichtung aneinander arretiert. Da sich der Schwerpunkt nunmehr näher an der Achse des Schwenklagers befindet, sind die durch das Gewicht erzeugten Drehmomente geringer, so dass zumindest ein teilweiser Abgleich erzielt ist, der in vielen Fällen ausreichen wird.

Weiter ist vorgesehen, dass der zweite Arm scheibenförmig oder abgewinkelt ausgebildet ist und ein zweites Schwenklager aufweist, über das er mit einem dritten Arm verbunden ist, der mit dem Mikroskop verbunden ist, wobei die Achse des Drehlagers und der ersten und zweiten Schwenklager im wesentlichen parallel zueinander und an den Ecken eines im wesentlichen rechtwinkligen Dreiecks mit der Achse des Drehlagers beim rechten Winkel angeordnet sind und wobei eine zweite Feststelleinrichtung zur winkelmäßigen Arretierung der zweiten und dritten Arme vorgesehen ist.

Bei dieser Ausführungsform wird nun, nachdem der eben beschriebene erste Abgleich durchgeführt ist, das zweite Schwenklager senkrecht über die Achse des Drehlagers gestellt. In dieser Stellung wird der zweite Arm festgehalten und die zweite Feststelleinrichtung gelöst. Der Schwerpunkt pendelt sich nun senkrecht zur ersten Einstellung unterhalb der Achse des Drehlagers ein. Anschließend werden dann mit der zweiten Feststelleinrichtung der zweite und dritte Arm aneinander arretiert. Nach diesen beiden Schritten befindet sich der Schwerpunkt sehr nahe bei der Drehachse, obwohl er genaugenommen bei der zweiten Pendelbewegung sich nicht direkt auf die Drehachse zu bewegt hat, sondern einen Kreisbogenabschnitt beschrieben hat, der unterhalb der Drehachse verläuft. Der Abstand ist aber nicht kritisch, wenn der dritte Arm genügend lang ist. Der restliche Abstand zwischen Schwerpunkt und Drehachse stört nicht mehr. Sollte dies doch der Fall sein, kann Schritt 1 (und ggf. Schritt 2) wiederholt werden, um so durch ein Iterationsverfahren den Schwerpunkt noch näher zur Drehachse zu bringen.

Die ersten und zweiten Feststelleinrichtungen können anstelle durch manuelle Betätigung auch durch elektrische oder elektromagnetische Aktoren gelöst oder arretiert werden. Wenn zusätzlich noch die Drehung um die Achse des Drehlagers durch einen ankuppelbaren motorischen Antrieb erfolgen kann, ist ein vollautomatischer Gewichtsausgleich möglich. In diesem Fall könnte das Operätionsmikroskop durch eine elektronische Steuerung in eine. Position gebracht werden, in der das erste Schwenklager über der Drehachse steht. Statt der Libelle würde ein Lagersensor diese Position an die Steuerung melden. Dann würde die ersten Feststelleinrichtung elektrisch gelöst und nach einigen Sekunden wieder arretiert werden. Anschließend erfolgt derselbe Vorgang für das andere Schwenklager. Dadurch könnte in sehr kurzer Zeit eine vollautomatische Balancierung durchgeführt werden.

Die Erfindung wird im Folgenden anhand einer vorteilhaften Ausführungsform unter Bezugnahme der auf die beigefügten Zeichnungen bspw. beschrieben. Es zeigen:
- Fig. 1: das Operationsmikroskop und die Aufhängung der Erfindung von der Seite;
- Fig. 2: einen horizontalen Schnitt durch die Anordnung, wobei die Schnittebene in die Strahlteilerebene gelegt ist; und
- Fig. 3 bis 6: aufeinanderfolgende Schritte des Gewichtsabgleichs.

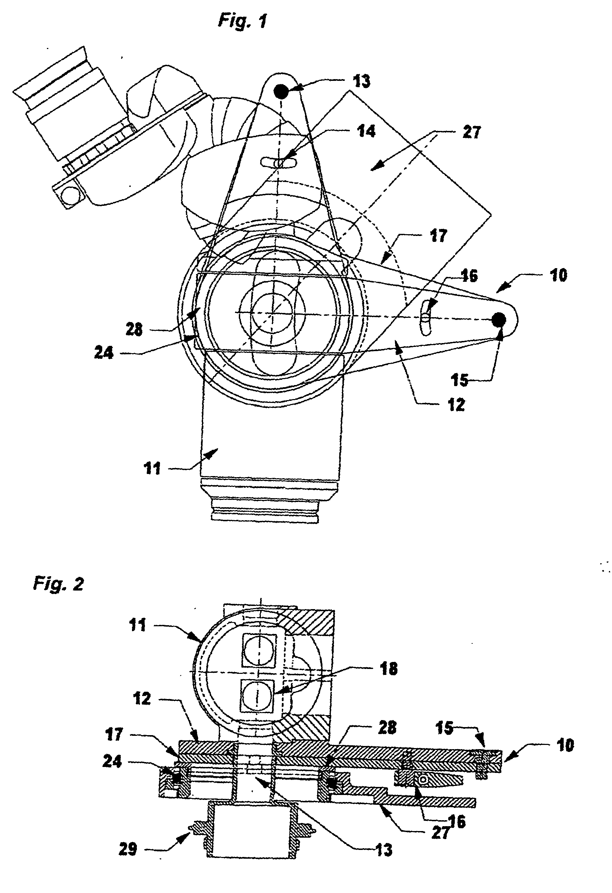

Wie dies in Fig. 1 und 2 gezeigt ist, ist eine aus drei Armen oder Scheiben 12, 17, 28 bestehende Anordnung 10 mit dem ersten Arm oder der ersten Scheibe 12 am Mikroskopkörper 11 befestigt. Über ein erstes Schwenklager 15 ist ein zweiter Arm oder eine zweite Scheibe 17 an dem ersten Arm 12 befestigt, so dass sich der zweite Arm 17 gegen den ersten Arm 12 verschwenken kann und dabei um das erste Schwenklager 15 schwenkt. Eine erste Feststelleinrichtung 16, die z. B. eine Klemmschraube aufweist, kann die Rotation der beiden Arme 12, 17 um das ersten Schwenklager 15 verhindern oder in einem festgelegten Winkelbereich ermöglichen.

In entsprechend gleichen Weise ist ein dritter Arm 28 öder dritte eine Scheibe 28 an der zweiten Scheibe 17 über ein zweites Schwenklager 13 und eine zweite Feststelleinrichtung 14 verbunden. An der dritten Scheibe 28 ist über ein Drehlager 24 der Aufhängearm 27 des Operationsmikroskops angebracht.

Die ersten und zweiten Schwenklager 13,15 müssen zusammen mit der Achse des Drehlagers 24 auf den Ecken eines Dreiecks angeordnet sein. Dabei ist dieses Dreieck im Idealfall ein rechtwinkliges Dreieck, wobei an der Ecke mit dem reichten Winkel die Achse des Drehlagers 24 angeordnet ist. Vom rechten Winkel ein wenig abweichende Winkel können aber je nach der konkreten Ausführungsform praktische Vorteile bieten.

Meist liegt bei Operationsmikroskopen mit einer raum- und gewichtssparenden konstruktiven Auslegung der Schwerpunkt nahe der Strahlteilerachse. Da am Strahlteiler 18 periphere Ausstattungsobjekte angebracht werden sollen, ist es zweckmäßig, die Anordnung 10 mit allen drei Armen 12, 17, 28 mit einer Öffnung auszuführen, die groß genug ist, dass ein Anschlussstück 29 für solche Objekte durchpasst und noch genügend Bewegungsmöglichkeiten für die Arme 12, 17, 28 verbleibt.

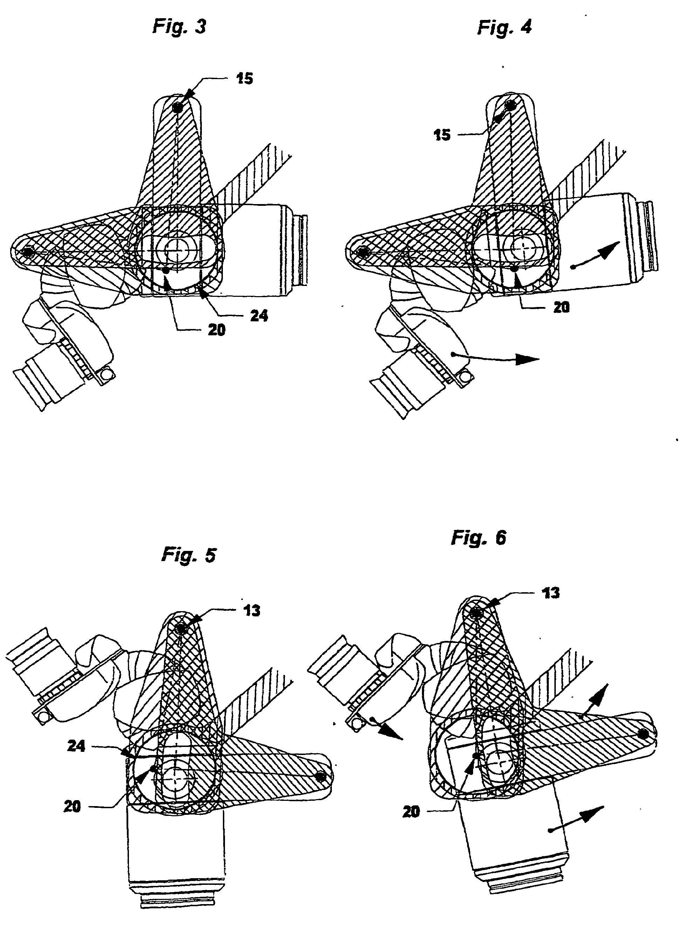

Der Gewichtsausgleich erfolgt nun, indem man das Operationsmikroskop um das Drehlager 24 so verschwenkt, dass die erste Schwenkachse 15 genau vertikal über der Achse des Drehlagers 24 steht (Fig. 3). Hierbei können Hilfsmittel, z. B. eine Wasserwaagenlibelle am zweiten Arm 17 nützlich sein. Der erste Arm 12 wird in dieser Stellung festgehalten, und die erste Feststelleinrichtung 16 wird gelöst. Dann pendelt sich der Mikroskopkörper so aus, dass der Schwerpunkt 20 näher zur Achse des Drehlagers 24 rückt, d. h., sich genau unter der Achse des Drehlagers 24 befindet (Fig. 4). In dieser Stellung wird die erste Feststelleinrichtung 16 wieder arretiert. Nun wird das Operationsmikroskop so gedreht, dass das zweite Schwenklager 13 über der Achse des Drehlagers 24 steht (Fig. 5). Es wird dann die zweite Feststelleinrichtung 14 gelöst und nach Auspendeln des Mikroskopkörpers wieder arretiert. Nun liegt der Schwerpunkt 20 sehr nahe bei der Achse des Drehlagers, und das Operationsmikroskop ist über den ganzen Neigungsbereich ausbalanciert (Fig. 6). Der Schwerpunkt 20 liegt allerdings nicht genau in der Achse des Drehlagers, sondern hat sich auf einem Kreisbogensegment, das unterhalb der Achse des Drehlagers 24 verläuft, bewegt. Falls der immer noch existierende Abstand von Schwerpunkt 20 und Achse des Drehlagers 24 zu groß ist, können die vorgenannten Schritte wiederholt werden.

Durch diesen Gewichtsausgleich ist eine schnelle und eindeutige Balancierung ohne großes Probieren möglich.

Ist durch die konstruktive Ausführung des Operationsmikroskops sichergestellt, dass sich bei An- oder Abbau peripherer Ausstattungsteile der Schwerpunkt in einer Richtung nur so wenig verschiebt, dass auf einen Gewichtsausgleich verzichtet werden kann, genügt eine aus zwei zueinander verschwenkbaren Armen oder Scheiben bestehende Anordnung. Vorzugsweise ist dann das Schwenklager zwischen den Armen auf oder nahe einer gedachten Linie angeordnet, die durch die Achse des Drehlagers gehend senkrecht verläuft zu dem ungefähr linienförmigen geometrischen Ort, den der Schwerpunkt einnehmen kann.

## Patentansprüche

1. Aufhängung für ein Operationsmikroskop mit einem Aufhängearm und einem Drehlager mit in wesentlichen horizontaler Drehachse, die einen ersten Arm (12) aufweist, der über das Drehlager (24) mit dem Aufhängearm (27) und über einen zweiten Arm (17), der mit dem ersten Arm (12) über ein erstes Schwenklager (15) verbunden ist, dessen Achse im wesentlichen parallel zur Drehachse ist, mit dem Operationsmikroskop (11) verbunden ist, wobei eine erste Feststelleinrichtung (16) zur winkelmäßigen Arretierung der Arme (12, 17) relativ zueinander vorgesehen ist, wobei der zweite Arm (17) ein zweites Schwenklager (13) aufweist, über das er mit einem dritten Arm (28) verbunden ist, der mit dem Mikroskop (11) verbunden ist, wobei die Achsen des Drehlagers (24) und der ersten und zweiten Schwenklager (15, 13) im wesentlichen parallel zueinander angeordnet sind und wobei eine zweite Feststelleinrichtung (14) zur winkelmäßigen Arretierung der zweiten und dritten Arme (17, 28) aneinander vorgesehen ist, **dadurch gekennzeichnet, dass** der zweite Arm derart scheibenförmig oder abgewinkelt ausgebildet ist, dass die Achsen des Drehlagers (24) und der ersten und zweiten Schwenklager (15, 13) an den Ecken eines im wesentlichen rechtwinkligen Dreiecks mit der Achse des Drehlagers (24) beim rechten Winkel angeordnet sind.

2. Aufhängung nach Anspruch 1, **dadurch gekennzeichnet, dass** die erste und/oder zweite Feststelleinrichtung (16, 14) eine Klemmschraube und einen Handhebel aufweist.

3. Aufhängung nach einem der Ansprüche 1 oder 2, **dadurch gekennzeichnet, dass** mindestens einer der Arme (12, 17, 28) eine Anzeigeeinrichtung aufweist, auf der ablesbar ist, ob sich das Schwenklager (15, 13) dieses Arms (12, 17, 28) vertikal über der Achse des Drehlagers (24) befindet.

4. Aufhängung nach Anspruch 3, **dadurch gekennzeichnet, dass** die Anzeigevorrichtung eine Wasserwaagenlibelle ist.

5. Aufhängung nach einem der Ansprüche 1 bis 4, **dadurch gekennzeichnet, dass** die Arme oder Scheiben (12, 17, 28) so ausgebildet sind, dass sie im Bereich des Strahlteilers des Operationsmikroskops durchbrochen sind und dass das Drehlager (24) diesen Bereich innen durchgängig umschließt.

6. Aufhängung nach einem der Ansprüche 1 bis 5, **dadurch gekennzeichnet, dass** die Feststelleinrichtungen (14, 16) elektrisch oder elektromagnetisch betätigbar sind.

7. Aufhängung nach einem der Ansprüche 1 bis 6, **dadurch gekennzeichnet, dass** ein motorischer Antrieb für die Drehung um die Achse des Drehlagers (24) vorgesehen ist.

## Claims

1. Suspension system for an operating microscope, with a suspension arm and with a rotary bearing having a substantially horizontal axis of rotation which exhibits a first arm (12) which is connected to the suspension arm (27) via the rotary bearing (24) and connected to the operating microscope (11) via a second arm (17) which is connected to the first arm (12) via a first swivel bearing (15), the axis of which is substantially parallel to the axis of rotation, wherein a first locking device (16) is provided for angular arresting of the arms (12, 17) relative to one another, wherein the second arm (17) exhibits a second swivel bearing (13), via which said second arm is connected to a third arm (28) which is connected to the microscope (11), wherein the axes of the rotary bearing (24) and of the first and second swivel bearings (15, 13) are arranged substantially parallel to one another and wherein a second locking device (14) is provided for angular arresting of the second and third arms (17, 28) on one another, **characterised in that** the second arm is of discoid or angled design in such a manner that the axes of the rotary bearing (24) and of the first and second swivel bearings (15, 13) at the vertices of a substantially right-angled triangle are arranged at a right angle with the axis of the rotary bearing (24).

2. Suspension system according to Claim 1, **characterised in that** the first and/or second locking devices (16, 14) exhibit(s) a clamping screw and a hand lever.

3. Suspension system according to one of Claims 1 or 2, **characterised in that** at least one of the arms (12, 17, 28) exhibits an indicating device on which it is readable whether the swivel bearing (15, 13) of this arm (12, 17, 28) is located vertically above the axis of the rotary bearing (24).

4. Suspension system according to Claim 3, **characterised in that** the indicating device is a spirit level.

5. Suspension system according to one of Claims 1 to 4, **characterised in that** the arms or discs (12, 17, 28) are designed in such a way that they are pierced in the region of the beam-splitter of the operating microscope and **in that** the rotary bearing (24) continuously encloses this region on the inside.

6. Suspension system according to one of Claims 1 to 5, **characterised in that** the locking devices (14, 16) are capable of being actuated electrically or electromagnetically.

7. Suspension system according to one of Claims 1 to 6, **characterised in that** a motorised drive is provided for the rotation about the axis of the rotary bearing (24).

## Revendications

1. Suspension pour un microscope chirurgical comprenant un bras de suspension et un palier rotatif avec un axe de rotation sensiblement horizontal, qui présente un premier bras (12), lequel est relié au bras de suspension (27) par le palier rotatif (24) et au microscope chirurgical (11) par un second bras (17) qui est relié au premier bras (12) par un premier palier pivotant (15), dont l'axe est sensiblement parallèle à l'axe de rotation, un premier dispositif de blocage (16) étant prévu pour le verrouillage en angle des bras (12, 17) les uns par rapport aux autres, le second bras (17) présentant un second palier pivotant (13) par lequel il est relié à un troisième bras (28), lequel est relié au microscope (11), les axes du palier rotatif (24) et des premiers et seconds paliers pivotants (15, 13) étant sensiblement parallèles entre eux et un second dispositif de blocage (14) étant prévu pour le verrouillage en angle des seconds et troisièmes bras (17, 28) les uns sur les autres, **caractérisée en ce que** le second bras est conçu en forme de disque ou coudé de telle sorte que les axes du palier rotatif (24) et des premiers et seconds paliers pivotants (15, 13) sont disposés sur les angles d'un triangle sensiblement rectangle avec l'axe du palier rotatif (24) sur l'angle droit.

2. Suspension selon la revendication 1, **caractérisée en ce que** le premier et/ou le second dispositif de blocage (16, 14) présente une vis de serrage et un levier manuel.

3. Suspension selon l'une quelconque des revendications 1 ou 2, **caractérisée en ce que** au moins l'un des bras (12, 17, 28) présente un dispositif d'affichage sur lequel on peut lire si le palier pivotant (15, 13) de ce bras (12, 17, 28) se trouve verticalement au-dessus de l'axe du palier rotatif (24).

4. Suspension selon la revendication 3, **caractérisée en ce que** le dispositif d'affichage est un niveau à bulle d'air.

5. Suspension selon l'une quelconque des revendications 1 à 4, **caractérisée en ce que** les bras ou disques (12, 17, 28) sont réalisés de telle sorte qu'ils sont traversés dans la zone du diviseur de faisceau du microscope chirurgical et **en ce que** le palier rotatif (24) entoure cette zone à l'intérieur de façon continue.

6. Suspension selon l'une quelconque des revendications 1 à 5, **caractérisée en ce que** les dispositifs de blocage (14, 16) peuvent être actionnés de façon électrique ou électromagnétique.

7. Suspension selon l'une quelconque des revendications 1 à 6, **caractérisée en ce qu'**un entraînement par moteur est prévu pour la rotation autour de l'axe du palier rotatif (24).
